(19) Europäisches Patentamt
European Patent Office
Office européen des brevets

(11) Veröffentlichungsnummer: **0 155 901 B1**

# (12) EUROPÄISCHE PATENTSCHRIFT

(45) Veröffentlichungstag der Patentschrift: **08.05.91**

(51) Int. Cl.5: **C07C 405/00**, A61K 31/557

(21) Anmeldenummer: **85730037.0**

(22) Anmeldetag: **06.03.85**

Die Akte enthält technische Angaben, die nach dem Eingang der Anmeldung eingereicht wurden und die nicht in dieser Patentschrift enthalten sind.

## (54) Neue Carbacycline, Verfahren zu ihrer Herstellung und ihre Verwendung als Arzneimittel.

(30) Priorität: **08.03.84 DE 3408699**

(43) Veröffentlichungstag der Anmeldung:
**25.09.85 Patentblatt 85/39**

(45) Bekanntmachung des Hinweises auf die Patenterteilung:
**08.05.91 Patentblatt 91/19**

(84) Benannte Vertragsstaaten:
**AT BE CH DE FR GB IT LI LU NL SE**

(56) Entgegenhaltungen:
**EP-A- 011 591**
**EP-A- 0 080 718**
**EP-A- 0 086 404**
**WO-A-83/04021**
**GB-A- 2 070 596**

(73) Patentinhaber: **SCHERING AKTIENGESELLSCHAFT Berlin und Bergkamen**
**Müllerstrasse 170/178 Postfach 65 03 11**
**W-1000 Berlin 65(DE)**

(72) Erfinder: **Skuballa, Werner, Dr.**
**Olwenstrasse 13**
**W-1000 Berlin 28(DE)**
Erfinder: **Radüchel, Bernd, Dr.**
**Gollanczstrasse 132**
**W-1000 Berlin 28(DE)**
Erfinder: **Vorbrüggen, Helmut, Prof. Dr.**
**Wilkestrasse 7**
**W-1000 Berlin 28(DE)**
Erfinder: **Schillinger, Ekkehard, Dr.**
**Im Amseltal 50**
**W-1000 Berlin 28(DE)**
Erfinder: **Stürzebecher, Claus-Steffen, Dr.**
**Kuckucksweg 6**
**W-1000 Berlin 33(DE)**

**EP 0 155 901 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue Carbacyclin-Derivate, Verfahren zu ihrer Herstellung sowie ihre Verwendung als Arzneimittel.

Der Vorläufer der Carbacycline, Prostacyclin, wurde 1976 isoliert und im selben Jahr in seiner Struktur aufgeklärt (Prostaglandins 12, 915, 1976). Als Prostaglandin-Kurzbezeichnung ist für Prostacyclin seit geraumer Zeit die Bezeichnung PG $I_2$ gebräuchlich. Entsprechend werden Carbacycline auch 6a-Carbaprostaglandine-$I_2$ genannt.

Die Nomenklatur der erfindungsgemäßen Verbindungen basiert auf einem Vorschlag von Morton und Brokaw (J. Org. Chem. 44, 2280 [1979]). Bei der Synthese dieser Verbindungen entstehen stets zwei Doppelbindungsisomere, die durch den Zusatz (5E) oder (5Z) charakterisiert werden. Substituiert man die obere Kette beispielsweise durch einen aromatischen Rest, erhält man Strukturformeln, die durch die folgenden Namen beschrieben werden können:

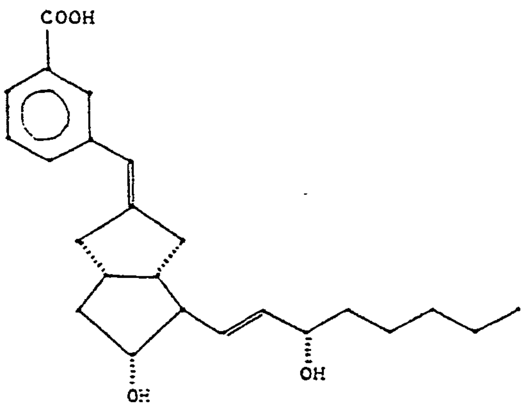

(5E)-2,3,4-Trinor-1,5-inter-m-phenylen-6a-carba-prostaglandin-$I_2$

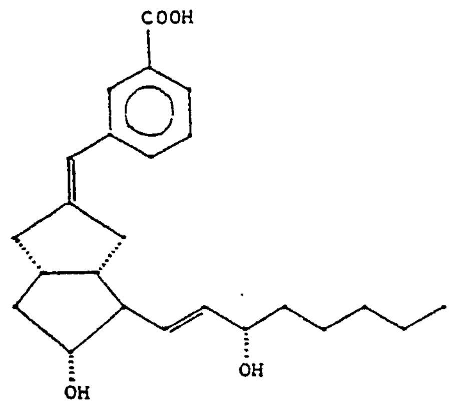

(5Z)-2,3,4-Trinor-1,5-inter-m-phenylen-6a-carba-prostaglandin-$I_2$

Derartige Prostacyclinanaloga sind bereits in der DE-A- 3 146 278 und EP-A- 0062 902 beschrieben.

Aus WO 83/04021 sind 2,3,4-Trinor-1,5-inter-m-phenylen-PG$7_2$-Derivate z.T. mit einer Dreifachbindung in 19,20-Stellung in der unteren Seitenkette bekannt. Diese Verbindungen besitzen ebenso wie die Interphenylencarbacycline aus GB 2070596, die in der unteren Kette eine Doppelbindung neben der in 13,14-Stellung tragen, Thrombozytenaggregationshemmende Wirkung. Darüberhinaus wirken die Verbindungen aus WO 83/04021 cytoprotektiv.

Auf Grund ihrer biologischen und pharmakologischen Eigenschaften sind Prostacycline und ihre Analoga zur Therapie und Prophylaxe von Thrombosen, Infarkten und anderer Herz-Kreislauf-Erkrankungen geeignet. Häufig ist die Wirkungsdauer dieser Verbindungen für therapeutische Zwecke noch zu kurz. Deshalb haben alle Strukturveränderungen an bekannten PG$I_2$-Derivaten das Ziel, die Wirkungsdauer zu verlängern, die Selektivität der Wirksamkeit zu steigern und gleichzeitig die Wirkungsdosis herabzusetzen.

Es wurde nun gefunden, daß durch Einführung einer Dreifachbindung in der 18,19- Position der unteren Kette der 2,3,4-Trinor-1,5-inter-m-phenylen-6a-carbaprostaglandin-$I_2$-Analoga die Wirksamkeit verbessert, die Selektivität erhöht und die Wirkungsdauer verlängert werden kann.

Die erfindungsgemäßen Verbindungen wirken blutdrucksenkend und bronchodilatatorisch. Sie sind außerdem zur Vasodilatation, Inhibierung der Thrombozytenaggregation und der Magensäuresekretion sowie zur Cytoprotektion an Magen, Herzen, Leber, Pankreas und Niere geeignet.

Die Erfindung betrifft Carbacyclinderivate der Formel I

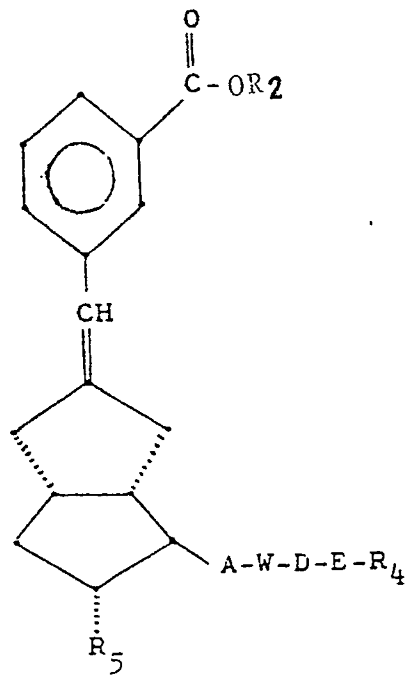

(I),

worin

- $R_2$ ein Wasserstoffatom oder einen $C_1$-$C_4$-Alkyl-Rest,
- A eine trans-CH = CH-, -C≡C-, oder -$CH_2$-$CH_2$-Gruppe,
- W eine Hydroxymethylengruppe, wobei die OH-Gruppe $\alpha$- oder $\beta$-ständig sein kann,
- D eine gegebenenfalls durch eine Alkylgruppe substituierte Ethylen Gruppe mit bis en 5 C-Atomen,
- E eine -C≡C-Gruppe,
- $R_4$ eine $C_1$-$C_7$-Alkyl-Gruppe,
- $R_5$ eine Hydroxygruppe, und deren Cyclodextrinclathrate und

falls $R_2$ die Bedeutung eines Wasserstoffatoms hat, deren Salze mit physiologisch verträglichen Basen bedeuten.

Die Verbindungen der Formel I stellen sowohl (5E)-als auch (5Z)-Isomere dar.

Als Alkylgruppen $R_2$ sind gerad- oder verzweigtkettige Alkylgruppen mit 1 - 4 C-Atomen zu betrachten, wie beispielsweise Methyl, Äthyl, Propyl, Isopropyl, Butyl, Isobutyl, tert.-Butyl.

Als bevorzugte Alkylgruppen $R_2$ sind solche mit 1 - 4 C-Atomen, wie z.B. Methyl, Äthyl, Propyl, Isobutyl, Butyl, zu nennen.

Als Alkylgruppe $R_4$ kommen gerad- und verzweigtkettige, gesättigte mit 1 - 7 C-Atomen, infrage. Beispielsweise genannt seien Methyl-, Äthyl-, Propyl-, Butyl-, Isobutyl-, tert.-Butyl-, Pentyl-, Hexyl-, Heptyl-.

Als D-Gruppe kommen beispielsweise: Äthylen, 1,2-Propylen, Äthyläthylen infrage.

Zur Salzbildung mit den freien Säuren ($R_2$ = H) sind anorganische und organische Basen geeignet, wie sie dem Fachmann zur Bildung physiologisch verträglicher Salze bekannt sind. Beispielsweise seien genannt: Alkalihydroxide, wie Natrium- und Kaliumhydroxid, Erdalkalihydroxide, wie Calciumhydroxid, Ammoniak, Amine, wie Äthanolamin, Diäthanolamin, Triäthanolamin, N-Methylglucamin, Morpholin- Tris-(hydroxymethyl)-methylamin usw.

Die Erfindung betrifft ferner ein Verfahren zur Herstellung der Verbindungen der Formel I, das dadurch gekennzeichnet ist, daß man eine Verbindung der Formel II

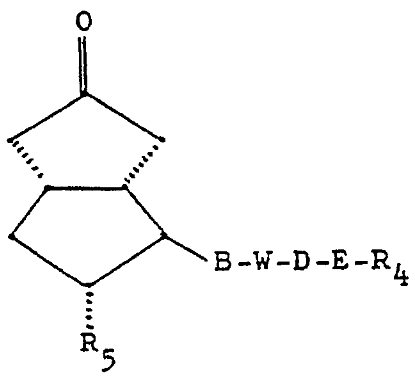

(II),

worin $R_5$, W, D, E und $R_4$ die obenangegebenen Bedeutungen aufweisen und B eine trans-Doppelbindung oder eine Dreifachbindung oder eine -CH = CBr-Gruppe bedeutet, gegebenenfalls nach Schutz anwesender freier Hydroxygruppen mit einem Wittig-Reagenz der Formel III

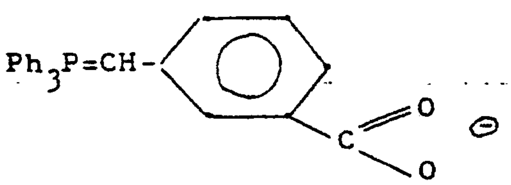

(III),

umsetzt und gegebenenfalls anschließend in beliebiger Reihenfolge Isomere trennt und/oder geschützte Hydroxygruppen freisetzt und/oder eine veresterte Carboxygruppe verseift oder eine Carboxygruppe mit einer physiologisch verträglichen Base in ein Salz überführt.

Die Umsetzung der Verbindung der allgemeinen Formel II mit dem Wittig-Reagenz der Formel III, das man aus dem entsprechenden Phosphoniumsalz mit Methansulfinylmethylnatrium oder Methansulfinylkalium oder Kalium-tert.-butylat in Dimethylsulfoxid oder Dimethylsulfoxid-Tetrahydrofurangemischen hergestellt, wird bei Temperaturen von 0 °C bis 100 °C, vorzugsweise 20 °C bis 60 °C, in einem aprotischen Lösungsmittel oder Lösungsmittelgemisch, vorzugsweise Dimethylsulfoxid, Dimethylformamid oder Tetrahydrofuran, vorgenommen. Die Trennung der dabei erhaltenen Z- und E-konfigurierten Olefine (5,6-Position) erfolgt auf übliche Art, beispielsweise durch Säulen- oder Schichtchromatographie. Bei der vorstehend beschriebenen Wittig-Olefinierung erfolgt, wenn B eine CH = CBr-Gruppe bedeutet, gleichzeitig unter Abspaltung von Bromwasserstoff die Bildung der 13,14-Acetylenbindung.

Die Verseifung der Prostaglandinester wird nach den dem Fachmann bekannten Methoden durchgeführt, wie beispielsweise mit basischen Katalysatoren.

Die Einführung der Gruppe -$OR_2$ bei welcher $R_2$ eine Alkylgruppe mit 1 - 4 C-Atomen darstellt, erfolgt nach den dem Fachmann bekannten Methoden. Die Carboxyverbindungen ($R_2$ = H) werden beispielsweise mit Diazokohlenwasserstoffen in an sich bekannter Weise umgesetzt. Die Veresterung mit Diazokohlenwasserstoffen erfolgt zum Beispiel dadurch, daß man eine Lösung des Diazokohlenwasserstoffes in einem inerten Lösungsmittel, vorzugsweise in Diäthyläther mit der Carboxyverbindung in dem gleichen oder in einem anderen inerten Lösungsmittel, wie zum Beispiel Methylenchlorid, vermischt. Nach beendeter Umsetzung in 1 bis 30 Minuten wird das Lösungsmittel entfernt und der Ester in üblicher Weise gereinigt. Diazoalkane sind entweder bekannt oder können nach bekannten Methoden hergestellt werden [Org. Reactions Bd. 8, Seiten 389 - 394 (1954)].

Die Prostaglandin-Derivate der allgemeinen Formel I mit $R_2$ in der Bedeutung eines Wasserstoffatoms können mit geeigneten Mengen der entsprechenden anorganischen Basen unter Neutralisierung in Salze überführt werden. Beispielsweise erhält man beim Lösen der entsprechenden PG-Säuren in Wasser, welches die stöchiometrische Menge der Base enthält, nach Abdampfen des Wassers oder nach Zugabe eines mit Wasser mischbaren Lösungsmittels, zum Beispiel Alkohol oder Aceton, das feste anorganische Salz.

Die Herstellung der Aminsalze erfolgt in üblicher Weise. Dazu wird die PG-Säure zum Beispiel in einem geeigneten Lösungsmittel, wie Äthanol, Aceton, Diäthyläther oder Benzol, gelöst und mindestens die

stöchiometrische Menge des Amins dieser Lösung zugesetzt. Dabei fällt das Salz gewöhnlich in fester Form an oder wird nach Verdampfen des Lösungsmittels in üblicher Weise isoliert.

Die Freisetzung einer funktionell abgewandelten OH-Gruppe zu den Verbindungen der allgemeinen Formel I erfolgt nach bekannten Methoden. Beispielsweise wird die Abspaltung von Ätherschutzgruppen in einer wäßrigen Lösung einer organischen Säure, wie zum Beispiel Essigsäure, Propionsäure u.a. oder in einer wäßrigen Lösung einer anorganischen Säure, wie zum Beispiel Salzsäure, durchgeführt. Zur Verbesserung der Löslichkeit wird zweckmäßigerweise ein mit Wasser mischbares inertes organisches Lösungsmittel zugesetzt. Geeignete organische Lösungsmittel sind zum Beispiel Alkohole, wie Methanol und Äthanol, und Äther, wie Dimethoxyäthan, Dioxan und Tetrahydrofuran. Tetrahydrofuran wird bevorzugt angewendet. Die Abspaltung wird vorzugsweise bei Temperaturen zwischen 20 °C und 80 °C durchgeführt.

Die Abspaltung der Silylätherschutzgruppen erfolgt beispielsweise mit Tetrabutylammoniumfluorid. Als Lösungsmittel sind beispielsweise geeignet Tetrahydrofuran, Diäthyläther, Dioxan, Methylenchlorid usw. Die Abspaltung wird vorzugsweise bei Temperaturen zwischen 0 °C und 80 °C durchgeführt.

Die Verseifung der Acylgruppen erfolgt beispielsweise mit Alkali- oder Erdalkalicarbonaten oder -hydroxyden in einem Alkohol oder der wäßrigen Lösung eines Alkohols. Als Alkohole kommen aliphatische Alkohole in Betracht, wie zum Beispiel Methanol, Äthanol, Butanol usw., vorzugsweise Methanol. Als Alkalicarbonate und -hydroxyde seien Kalium- und Natriumsalze genannt, bevorzugt sind jedoch die Kaliumsalze. Als Erdalkalicarbonate und -hydroxyde sind beispielsweise geeignet Calciumcarbonat, Calciumhydroxyd und Bariumcarbonat. Die Umsetzung erfolgt bei -10 °C bis 70 °C, vorzugsweise bei 25 °C.

Die als Ausgangsmaterial dienenden Verbindungen der Formel II können, sofern sie nicht bekannt sind, nach den in den DE-A- 28 45 770, 30 48 906, 31 21 155, 32 04 443, 32 09 702, 32 25 288, 32 26 550, 32 37 200, 33 06 123 und 33 06 125 beschriebenen Verfahren hergestellt werden.

Die 1-Carboxy-Verbindungen und die anderen $COOR_2$-Verbindungen dieser Erfindung wirken blutdrucksenkend und bronchodilatorisch. Sie sind weiterhin geeignet zur Hemmung der Thrombozyten-Aggregation. Die 1-Keto-Verbindungen dieser Erfindung wirken selektiv cytoprotektiv am Magen, Darm, Herz, an der Leber, Niere und am Pankreas. Folglich stellen die neuen Carbacyclin-Derivate der Formel I wertvolle pharmazeutische Wirkstoffe dar. Darüber hinaus weisen sie bei ähnlichem Wirkungsspektrum, verglichen mit entsprechenden Prostaglandinen, eine höhere Spezifität und vor allem eine wesentlich längere Wirksamkeit auf. Im Vergleich zu $PGI_2$ zeichnen sie sich durch größere Stabilität aus. Die hohe Gewebsspezifität der neuen Prostaglandine zeigt sich bei der Untersuchung an glattmuskulären Organen, wie zum Beispiel am Meerschweinchenileum oder an der isolierten Kaninchentrachea, wo eine wesentlich geringere Stimulation zu beobachten ist als bei der Applikation natürlicher Prostaglandine vom E-, A- oder F-Typ.

Die neuen Carbacyclin-Analoga besitzen die für Prostacycline typischen Eigenschaften, wie zum Beispiel Senkung des peripheren arteriellen und koronaren vaskulären Widerstandes, Inhibierung der Thrombozytenaggregation und Auflösung von Plättchenthromben, myocardiale Zytoprotektion und damit Senkung des systemischen Blutdruckes ohne zugleich Schlagvolumen und koronare Durchblutung zu senken; Behandlung von Schlaganfall, Prophylaxe und Therapie koronarer Herzerkrankungen, koronarer Thrombose, des Herzinfarktes, peripherer Arterienerkrankungen, Arteriosklerose und Thrombose, Prophylaxe und Therapie ischaemischer Attacken des ZNS-Systems, Therapie des Schocks, Inhibierung der Bronchokonstriktion, Inhibierung der Magensäuresekretion, Zytoprotektion der Magen- und Darmschleimhaut, Zytoprotektion in der Leber, Niere und im Pankreas, antiallergische Eigenschaften, Senkung des pulmonalen vaskulären Widerstandes und des pulmonalen Blutdruckes, Förderung der Nierendurchblutung, Anwendung anstelle von Heparin oder als Adjuvans bei der Dialyse der Hämofiltration, Konservierung von Blutplasmakonserven, besonders von Blutplättchenkonserven, Inhibierung von Geburtswehen, Behandlung von Schwangerschaftstoxikose, Erhöhung der zerebralen Durchblutung etc. Außerdem besitzen die neuen Carbacyclinderivate antiproliferative und antidiarrhoegene Eigenschaften.
Mit den neuen Carbacyclinen können auch Organe vor ihrer Transplantation behandelt werden.

Die Carbacycline dieser Erfindung können auch in Kombination, zum Beispiel mit ß-Blockern, Diuretika, Phosphodiesterasehemmern, Calciumantagonisten, nichtsteroidalen Entzündungshemmern, Leukotriensynthesehemmern, Leukotrienantagonisten, Thromboxansynthesehemmern oder Thromboxanantagonisten verwendet werden.

Die Dosis der Verbindungen ist 1 - 1500 µg/kg/Tag, wenn sie am menschlichen Patienten verabreicht werden. Die Einheitsdosis für den pharmazeutisch akzeptablen Träger beträgt 0,01 - 100 mg.

Bei intravenöser Injektion an wachen, hypertonen Ratten in Dosen von 5, 20 und 100 µg/kg Körpergewicht zeigen die erfindungsgemäßen Verbindungen eine stärkere blutdrucksenkende und länger anhaltende Wirkung als $PGE_2$ und $PGA_2$, ohne wie $PGE_2$ Durchfälle oder $PGA_2$ kardiale Arrhythmien auszulösen.

Bei intravenöser Injektion an narkotisierten Kaninchen zeigen die erfindungsgemäßen Verbindungen im

Vergleich zu $PGE_2$ und $PGA_2$ eine stärkere und erheblich länger anhaltende Blutdrucksenkung, ohne daß andere glattmuskuläre Organe oder Organfunktionen beeinflußt werden.

Für die parenterale Verabreichung werden sterile, injizierbare, wäßrige oder ölige Lösungen benutzt. Für die orale Applikation sind beispielsweise Tabletten, Dragees oder Kapseln geeignet.

Die Erfindung betrifft damit auch Arzneimittel auf Basis der Verbindungen der allgemeinen Formel I und üblicher Hilfs- und Trägerstoffe einschließlich Cyclodextrinclathrate.

Die erfindungsgemäßen Wirkstoffe sollen in Verbindung mit den in der Galenik bekannten und üblichen Hilfsstoffen zum Beispiel zur Herstellung von Blutdrucksenkern, Thrombocytenaggregationshemmern oder Cytoprotektiva dienen.

## Beispiel 1

(5E)-(16RS)-16-Methyl-18,18,19,19-tetradehydro-2,3,4-trinor-1,5-inter-m-phenylen-6a-carba-prostaglandin-$I_2$

Zu einer Lösung von 10 g Triphenyl-3-carboxybenzyl-phosphoniumbromid in 24 ml Dimethylsulfoxid und 14 ml Tetrahydrofuran gibt man bei 5 °C innerhalb von 45 Minuten 4,48 g Kalium-tert.-butylat und rührt noch 45 Minuten bei 5 °C. Zur roten Ylenlösung fügt man eine Lösung von 1,8 g (1R,5S,6R,7R)-7-(Tetrahydropyran-2-yloxy)-6-[(E)-(3S,4RS)-4-methyl-3-(tetrahydropyran-2-yloxy)-oct-1-en-6-inyl]-bicyclo-[3.3.0]-octan-3-on in 16 ml Tetrahydrofuran und rührt 18 Stunden bei 35 °C. Das Reaktionsgemisch wird auf Eiswasser gegossen, mit Zitronensäure auf pH5 angesäuert und mit Methylenchlorid extrahiert. Die organische Phase wird mit Sole geschüttelt, über Magnesiumsulfat getrocknet und im Vakuum eingedampft. Den Rückstand reinigt man durch mehrfache Chromatographie an Kieselgel. Mit Hexan/Essigester (6 ÷ 4) erhält man zunächst 390 mg des Z-konfigurierten Olefins, 0,9 g ungetrenntes 5E/Z-Gemisch sowie als polarere Komponente 370 mg (5E)-(16RS)-16-Methyl-18,18,19,19-tetradehydro-2,3,4-trinor-1,5-inter-m-phenylen-6a-carba-prostaglandin-$I_2$-11,15-bis-(tetrahydropyranyläther) als farbloses Öl.

IR ($CHCl_3$): 3605, 3400 (breit), 2950, 2868, 1695 (breit), 1600, 1573, 1380, 974/cm.

Zur Schutzgruppenabspaltung rührt man 370 mg des vorstehend erhaltenen Produkts mit 40 ml einer Mischung aus Essigsäure/Wasser/Tetrahydrofuran (65/35/10) 20 Stunden bei 25 °C. Anschließend dampft man im Vakuum unter Zusatz von Toluol ein und chromatographiert den Rückstand an Kieselgel. Mit Methylenchlorid/Isopropanol (9 + 1) erhält man 205 mg der Titelverbindung als farbloses Öl.

IR: 3605, 3400 (breit), 2960, 2920, 1695 (breit), 1600, 1582, 970/cm.

## Beispiel 2

(5Z)-(16RS)-16-Methyl-18,18,19,19-tetradehydro-2,3,4-trinor-1,5-inter-m-phenylen-6a-carba-prostaglandin-$I_2$

390 mg des nach chromatographischer Trennung in Beispiel 1 erhaltenen (5Z)-konfigurierten Olefins rührt man 18 Stunden bei 25 °C mit 40 ml einer Mischung aus Essigsäure/Wasser/Tetrahydrofuran (65/35/10). Man dampft im Vakuum unter Zusatz von Toluol ein und chromatographiert den Rückstand an Kieselgel. Mit Methylenchlorid/Isopropanol (9 + 1) erhält man 220 mg der Titelverbindung als Öl.

IR: 3605, 3400 (breit), 2960, 2920, 1730, 1695, 1600, 1581, 970/cm.

## Beispiel 3

(5E)-(16RS)-16,20-Dimethyl-18,18,19,19-tetradehydro-2,3,4-trinor-1,5-inter-m-phenylen-6a-carba-prostaglandin-$I_2$

In Analogie zu Beispiel 1 erhält man aus 1,6 g (1R,5S,6R,7R)-7-(Tetrahydropyran-2-yloxy)-6-[(E)-(3S,4RS)-4-methyl-3-(tetrahydropyran-2-yloxy)-non-1-en-6-inyl]-bicyclo[3.3.0] octan-3-on 420 mg (5E)-(16RS)-16,16-Dimethyl-18,18,19,19-tetradehydro-2,3,4-trinor-1,5-inter-m-phenylen-6a-carba-prostaglandin-$I_2$-11,15-bis(tetrahydropyranyläther) als farbloses Öl.

IR: 3600, 3410 (breit), 2950, 2869, 1696 (breit), 1600, 1573, 975/cm.

Nach Abspaltung der Schutzgruppen gemäß Beispiel 1 erhält man 280 mg der Titelverbindung als farbloses Öl.

IR: 3600, 3380 (breit), 2962, 2922, 1696 (breit), 1600, 1573, 971/cm.

## Beispiel 4

(5E)-18,18,19,19-Tetradehydro-16,16,20-trimethyl-2,3,4-trinor-1,5-inter-m-phenylen-6a-carba-prostaglandin-$I_2$

In Analogie zu Beispiel 1 erhält man aus 1,4 g (1R,5S,6R,7R)-7-(Tetrahydropyran-2-yloxy)-6-[(E)-(3R)-4,4-dimethyl-3-(tetrahydro-pyran-2-yloxy)-non-1-en-6-inyl]-bicyclo[3.3.0]octan-3-on 390 mg (5E)-18,18,19,19-Tetradehydro-16,16,20-trimethyl-2,3,4-trinor-1,5-inter-m-Phenylen-6a-carba-prostaglandin-$I_2$-11,15-bis-(tetrahydropyranyläther) als farbloses Öl.

IR: 3600, 3400 (breit), 2960, 2925, 1697 (breit), 1600, 1574, 972/cm.

## Beispiel 5

(5E)-(16RS)-13,14-Didehydro-16-methyl-18,18,19,19-tetradehydro-2,3,4-trinor-1,5-inter-m-phenylen-6a-carba-prostaglandin-$I_2$

In Analogie zu Beispiel 1 erhält man aus 1,50 g (1R,5S,6R,7R)-7-(Tetrahydropyran-2-yloxy)-6-[(3S,4RS)-4-methyl-3-(tetrahydropyran-2-yloxy)-octa-1,6-diinyl]-bicyclo[3.3.0]octan-3-on 460 mg (5E)-(16RS)-13,14-Didehydro-16-methyl-18,18,19,19-tetradehydro-2,3,4-trinor-1,5-inter-m-phenylen-6a-carba-prostaglandin-$I_2$-11,15-bis(tetrahydropyranyläther) als farbloses Öl.

IR: 3600, 3405 (breit), 2955, 2877, 2215, 1698 (breit), 1600, 1581/cm.

Nach Abspaltung der Schutzgruppen gemäß Beispiel 1 erhält man 251 mg der Titelverbindung als farbloses Öl.

IR: 3600, 3400 (breit), 2961, 2928, 2212, 1698 (breit), 1601, 1579/cm.

## Beispiel 6

(5E)-(16S)-13,14-Didehydro-16,20-dimethyl-18,18,19,19-tetradehydro-2,3,4-trinor-1,5-inter-m-phenylen-6a-carba-prostaglandin-$I_2$

In Analogie zu Beispiel 1 erhält man aus 2,0 g (1R,5S,6R,7R)-7-(Tetrahydropyran-2-yloxy)-6-[3S,4S)-4-methyl-3-(tetrahydropyran-2-yloxy)-nona-1,6-diinyl]-bicyclo [3.3.0]octan-3-on 590 mg (5E)-(16S)-13,14-Didehydro-16,20-dimethyl-18,18,19,19-tetradehydro-2,3,4-trinor-1,5-inter-m-phenylen-6a-carba-prostaglandin-$I_2$-11,15-bis-(tetrahydropyranyläther) als farbloses Öl.

IR: 3600, 3400 (breit), 2958, 2875, 2230, 1695 (breit), 1600, 1582/cm.

Nach Abspaltung der Schutzgruppen gemäß Beispiel 1 erhält man 280 mg der Titelverbindung als farbloses Öl.

IR: 3600, 3400 (breit), 2960, 2924, 2230, 1696 (breit), 1600, 1579/cm.

## Beispiel 7

(5E)-(16RS)-16-Methyl-18,18,19,19-tetradehydro-2,3,4-trinor-1,5-inter-m-phenylen-6a-carba-prostaglandin-$I_2$-methylester

Zu einer Lösung von 150 mg (5E)-(16RS)-16-Methyl-18,18,19,19-tetradehydro-2,3,4-trinor-1,5-m-inter-phenylen-6a-carba-prostaglandin-$I_2$ in 10 ml Dichlormethan tropft man bei 0 °C eine etherische Lösung von Diazomethan bis zur bleibenden Gelbfärbung. Anschließend dampft man im Vakuum ein und chromatographiert den Rückstand an Kieselgel mit Ethylacetat/Hexan (8+2) und erhält 115 mg der Titelverbindung als farbloses Öl.

IR: 3600, 2955, 2864, 1710, 1601, 1578, 975/cm.

Beispiel 8

(5E)-(16RS)-16-Methyl-18,18,19,19-tetradehydro-2,3,4-trinor-1,5-inter-m-phenylen-6a-carba-prostaglandin-$I_2$-tris-(hydroxymethyl)-aminomethansalz

Zu einer Lösung von 185 mg (5E)-(16RS)-16-Methyl-18,18,19,19-tetradehydro-2,3,4-trinor-1,5-m-inter-phenylen-6a-carba-prostaglandin-$I_2$ in 40 ml Acetonitril fügt man bei 70 °C eine Lösung von 60 mg Tris-(hydroxymethyl)-aminomethan in 0,2 ml Wasser. Man läßt unter Rühren abkühlen, dekantiert nach 16 Stunden vom Lösungsmittel und trocknet den Rückstand im Vakuum. Man erhält 159 mg der Titelverbindung als wachsartige Masse.

## Ansprüche

**1.** Carbacyclinderivate der Formel I

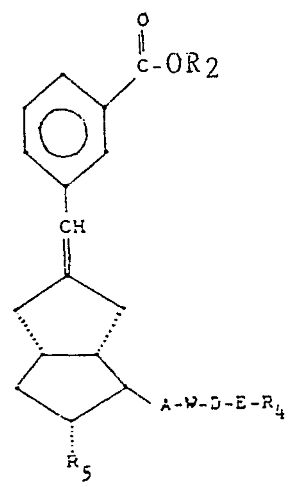

(I),

worin
$R_2$ ein Wasserstoffatom oder einen $C_1$-$C_4$-Alkyl-Rest,
A eine trans-CH=CH-, -C≡C-, oder -$CH_2$-$CH_2$-Gruppe,
W eine Hydroxymethylengruppe, wobei die OH-Gruppe α- oder β-ständig sein kann,
D eine gegebenenfalls durch ein Alkylgruppe Substituierte Ethylen-Gruppe mit bis zu 5 C-Atomen
E eine -C≡C-Gruppe,
$R_4$ eine $C_1$-$C_7$-Alkylgruppe,
$R_5$ eine Hydroxygruppe, und deren Cyclodextrinclathrate und
falls $R_2$ die Bedeutung eines Wasserstoffatoms hat, deren Salze mit physiologisch verträglichen Basen bedeuten.

**2.** Verfahren zur Herstellung der Verbindungen der Formel I, dadurch gekennzeichnet, daß man eine Verbindung der Formel II

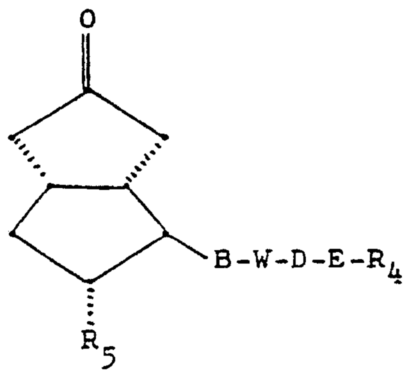

(II),

worin $R_5$, W, D, E und $R_4$ die obenangegebenen Bedeutungen aufweisen und B eine trans-Doppelbindung oder eine Dreifachbindung oder eine -CH = CBr-Gruppe bedeutet, gegebenenfalls nach Schutz anwesender freier Hydroxygruppen mit einem Wittig-Reagenz der Formel III

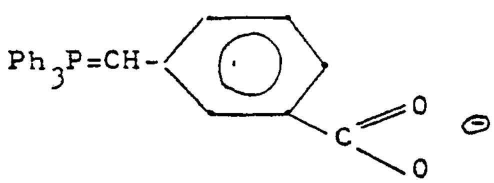

(III),

umsetzt und gegebenenfalls anschließend in beliebiger Reihenfolge Isomere trennt und/oder geschützte Hydroxygruppen freisetzt und/oder eine veresterte Carboxygruppe verseift oder eine Carboxygruppe mit einer physiologisch verträglichen Base in ein Salz überführt.

3. Arzneimittel, bestehend aus einer oder mehreren Verbindungen der Formel I und üblichen Hilfs- und Trägerstoffen.

4. (5E)-(16RS)-16-Methyl-18,18,19,19-tetradehydro-2,3,4-trinor-1,5-inter-m-phenylen-6a-carba-prostaglandin-$I_2$.

5. (5E)-(16S)-13,14-Didehydro-16,20-dimethyl-18,18,19,19-tetradehydro-2,3,4-trinor-1,5-inter-m-phenylen-6a-carba-prostaglandin-$I_2$.

## Claims

1. Carbacyclin derivatives of the formula I

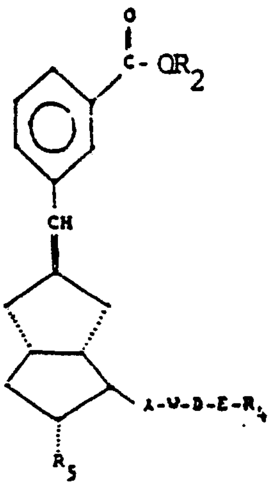

(I)

in which

- $R_2$ represents a hydrogen atom or a $C_1$-$C_4$-alkyl radical,
- A represents a trans-CH = CH-, -C≡C- or $-CH_2-CH_2-$ group,
- W represents a hydroxymethylene group, it being possible for the OH group to be in the α- or β-configuration,
- D represents an ethylene group having up to 5 carbon atoms which is optionally substituted by an alkyl group,
- E represents a -C≡C- group,
- $R_4$ represents a $C_1$-$C_7$-alkyl group,
- $R_5$ represents a hydroxy group, and the cyclodextrin clathrates thereof, and, if $R_2$ represents a hydrogen atom, the salts thereof with physiologically tolerable bases.

2. Process for the preparation of the compounds of the formula I, characterised in that a compound of the formula II

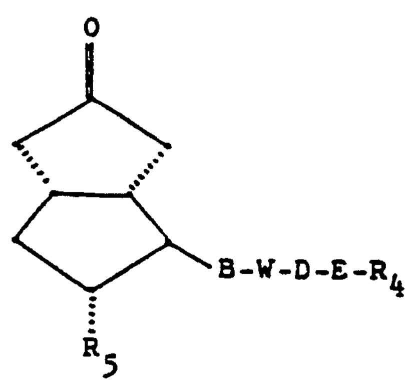

(II)

in which $R_5$, W, D, E and $R_4$ have the meanings given above and B represents a trans-double bond or a triple bond or a -CH = CBr- group, is reacted, optionally after protecting any free hydroxy groups present, with a Wittig reagent of the formula III

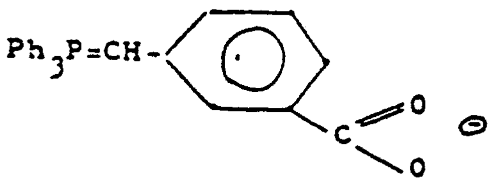

(III)

and then, optionally, in any sequence, isomers are separated and/or protected hydroxy groups are freed and/or an esterified carboxy group is hydrolysed or a carboxy group is converted into a salt with a physiologically tolerable base.

**3.** Medicament, consisting of one or more compounds of the formula I and customary adjuvants and carriers.

**4.** (5E)-(16RS)-16-methyl-18,18,19,19-tetradehydro-2,3,4-trinor-1,5-inter-m-phenylene-6a-carba-prostaglandin-$I_2$.

**5.** (5E)-(16S)-13,14-didehydro-16,20-dimethyl-18,18,19,19-tetradehydro-2,3,4-trinor-1,5-inter-m-phenylene-6a-carba-prostaglandin-$I_2$.

## Revendications

**1.** Les carbacyclines de formule I

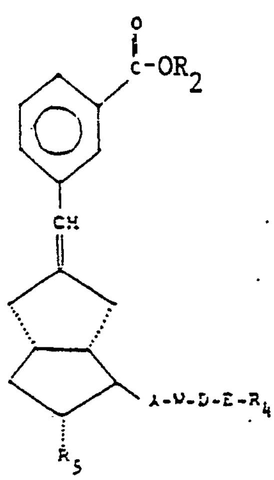

dans laquelle

- $R_2$ désigne un atome d'hydrogène ou un alkyle en $C_1$-$C_4$,
- A un groupe trans-CH=CH-, -C≡C- ou $-CH_2-CH_2-$,
- W un groupe hydroxyméthylène dont l'hydroxyle peut être à la position $\alpha$ ou $\beta$,
- D un groupe éthylène éventuellement alkylé pouvant avoir jusqu'à 5 atomes de carbone,
- E un groupe -C≡C-,
- $R_4$ un alkyle en $C_1$-$C_7$, et
- $R_5$ un hydroxyle, ainsi que leurs clathrates formés avec la cyclodextrine et, si $R_2$ est un atome d'hydrogène, leurs sels avec des bases compatibles du point de vue physiologique.

**2.** Procédé de préparation des composés de formule I de la revendication 1, procédé caractérisé en ce

que l'on fait réagir un composé de formule II

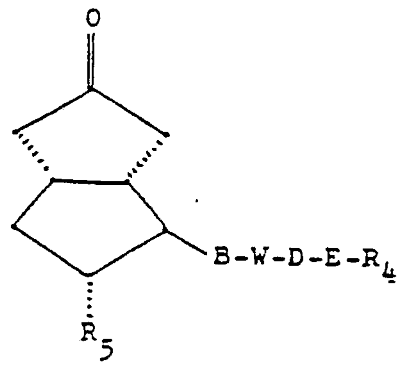

(II),

dans laquelle $R_5$, W, D, E et $R_4$ ont les significations qui leur ont été données ci-dessus et B désigne une double liaison en position trans ou une triple liaison ou encore un groupe -CH = CBr-, éventuellement après avoir eu protégé des groupes hydroxyliques libres présents, avec un réactif de Wittig de formule III

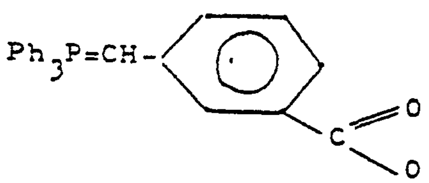

(III),

puis le cas échéant, et en procédant dans un ordre quelconque, on sépare des isomères et/ou on libère des groupes hydroxyliques protégés et/ou on saponifie un groupe carboxylique estérifié ou encore on salifie un groupe carboxylique avec une base compatible du point de vue physiologique.

**3.** Médicament comprenant un ou plusieurs composés de formule I de la revendication 1 avec des adjuvants et véhicules courants.

**4.** (5E)-(16RS)-16-Méthyl-18,18,19,19-tétradéhydro-2,3,4-trinor-1,5-inter-m-phénylène-6a-carba-prostaglandine-$I_2$.

**5.** (5E)-(16S)-13,14-Didéhydro-16,20-diméthyl-18,18,19,19-tétradéhydro-2,3,4-trinor-1,5-inter-m-phénylène-6a-carba-prostaglandine-$I_2$.